# EUROPEAN PATENT SPECIFICATION

(11) **EP 0 295 286 B1**
(45) Date of publication and mention of the grant of the patent: **08.03.1995**
(21) Application number: 88900597.1
(22) Date of filing: 28.12.1987
(51) Int. Cl.: C12N 15/00, C12P 21/00, C12R 1/19

(54) **METHOD FOR THE PRODUCTION OF SALMON GROWTH HORMONE USING A SYNTHETIC GENE**
VERFAHREN ZUR HERSTELLUNG EINES LACHSWACHSTUMSHORMONS MITTELS EINES KUNSTGENS
PROCEDE DE PRODUCTION DE L'HORMONE DE CROISSANCE DU SAUMON PAR L'UTILISATION D'UN GENE SYNTHETIQUE

(30) Priority: 31.12.1986 KR 1170886; 31.12.1986 KR 1170986
(43) Date of publication of application: 21.12.1988
(73) Proprietor: LUCKY, LTD., Seoul 150 (KR)
(72) Inventor: CHO, Joong, Myung, Seoul 133 (KR); LEE, Tae, Ho, Daejeon-shi Chungcheongnam-do 300-31 (KR); CHUNG, Hyun, Ho, Kwanak-gu Seoul 151 (KR); LEE, Yong, Beom, Daejeon-shi Chungcheongnam-do 300-01 (KR); LEE, Tae, Gyu, Seoul 132 (KR); PARK, Young, Woo, Daejeon-shi Chungcheongnam-do 300-31 (KR); HAN, Kyu, Beom, Daejeon-shi Chungcheongnam-do 300-31 (KR)
(74) Representative: Perry, Robert Edward
(86) International application number: KR8700014
(87) International publication number: WO8805079

(56) References cited:
- EP-A- 0 166 444
- EP-A- 0 209 068
- Journal of Biological Chemistry 259(10), 6311-6317, 1984, E. Jay et al.
- Nature 292, 756-762, 1981, M.D. Edge et al.
- Gene 39, 117-120, 1985, T. Tokunaga et al.

## Description

### Field of the Invention

The present invention relates to a method for the production of salmon growth hormone (SGH) using a synthetic gene, in yeast.

### Background of the Invention

High protein content feed or hormonal steroids have been added to the feed used at fish farms, in order to elevate the efficiency of the feed. It has been found that the steroids, however, are not metabolised immediately but remain in the fish for a long time after ingestion. Their presence is detrimental to those who eat the fish, and their use is increasingly subject to prohibition.

By contrast, SGH does not remain inside the body after ingestion. Because it has a species-specificity, as it is a protein of salmon itself, SGH is the most desirable material capable of increasing the feed efficiency.

Tokunaga et al, Gene 39:117-120 (1985), disclose the expression of a synthetic human growth hormone (hGH) gene under the control of the repressible acid phosphatase promoter, in yeast. After synthesis and cloning of the hGH gene into a vector for E. coli, the synthetic hGH gene was inserted into an E. coli-yeast shuttle vector, and the resultant vector was subsequently used for expression in yeast cells. The hGH gene was expressed at a level of 1.4 to 4.7 x 10⁶ molecules per cell, which is comparable to maximal level of expression of these genes.

### Summary of the Invention

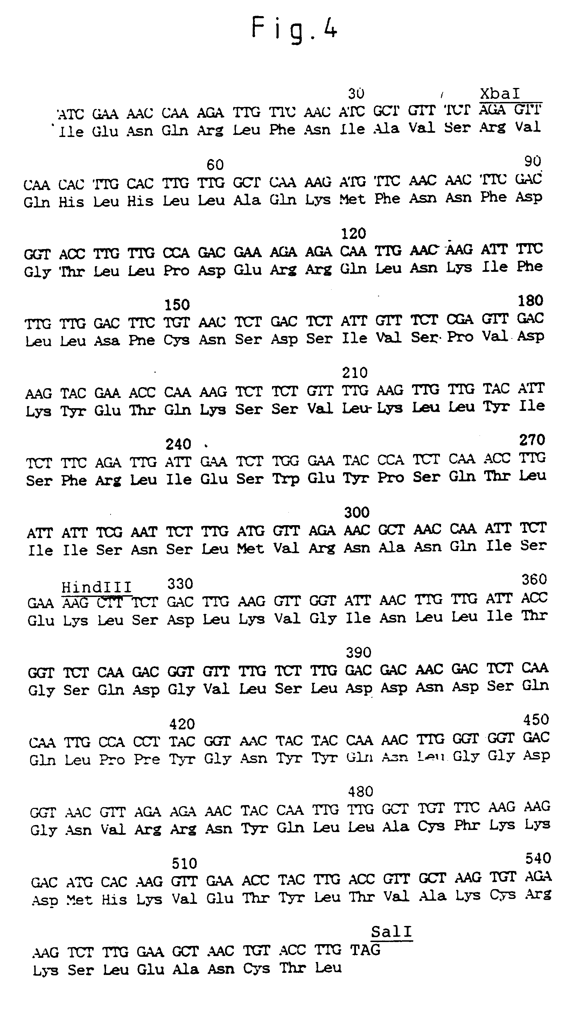

According to the present invention, SGH is produced by a method comprising (a) synthesising oligonucleotides having XbaI, HindIII and SalI restriction sites as shown in Fig. 4 and corresponding to the amino-acid sequence of salmon growth hormone, (b) restricting the oligonucleotides to form XbaI/HindIII and HindIII/SalI fragments, (c) ligating the fragments into respective vectors for E. coli, (d) isolating the said fragments from the respective vectors, (e) ligating the fragments to a vector for E. coli, to make a partial salmon growth hormone gene, lacking a portion of the N-terminus, (f) inserting the partial salmon growth hormone gene and a N-terminal synthetic adaptor into a vector for E. coli consisting of a hybrid alcohol dehydrogenase II/glyceraldehyde 3'-phosphate dehydrogenase promoter and a terminator, to obtain a cassette of promoter-salmon growth hormone gene-terminator, (g) inserting the cassette into an E. coli-yeast shuttle vector for expression in yeast, and (h) expressing the resultant vector in yeast cells.

The N-terminal synthetic adaptor is the following nucleotide sequence having NcoI site and XbaI site: 
5' - CATGATCGAAAACCAAAGATTGTTCAACATCGCTGTTT - 3'
3' - TAGCTTTTGGTTTCTAACAAGTTGTAGCGACAAAGATC - 5'
NcoI site
XbaI site
The yeast expression vector is named as pC1/1-SGH, containing the cassette comprising promoter-salmon growth hormone gene with an N-terminal synthetic adaptor-terminator, a complete 2 micron, a Leu 2d gene and an origin of replication.

For a better understanding of the invention, reference is made to the accompanying drawings and descriptive matter in which a preferred embodiment of the invention is illustrated.

### BRIEF DESCRIPTION OF THE DRAWINGS

Fig. 1 shows synthetic oligonucleotides corresponding to a salmon growth hormone gene and represented by a base sequence from the 5'-end to the 3'-end.

Fig.2 is a ligation strategy of oligonucleotides in accordance with Fig.1.

Fig.3 is a schematic cloning process of synthetic oligonucleotides to the plasmid, pUC18.

Fig.4 is the base sequence and putative amino acid sequence of confirmed salmon growth hormone gene.

Fig.5 is a cloning process into a yeast expression vector (pC1/1) for producing salmon growth hormone in yeast.

### DETAILED DESCRIPTION OF A PREFERRED EMBODIMENT

The present inventors selected the base sequence of salmon growth hormone with amino acid codons preferentially used by yeast cells, based upon the amino acid sequence of salmon growth hormone, reported by Sekine et al.[Proc. Natl.Acad. Sci,USA 82(1985) 4308] and the complete gene of mature salmon growth hormone was chemically synthesized using a DNA synthesizer(Applied Biosystem Model 380B, USA) according to the phosphoramidate method.

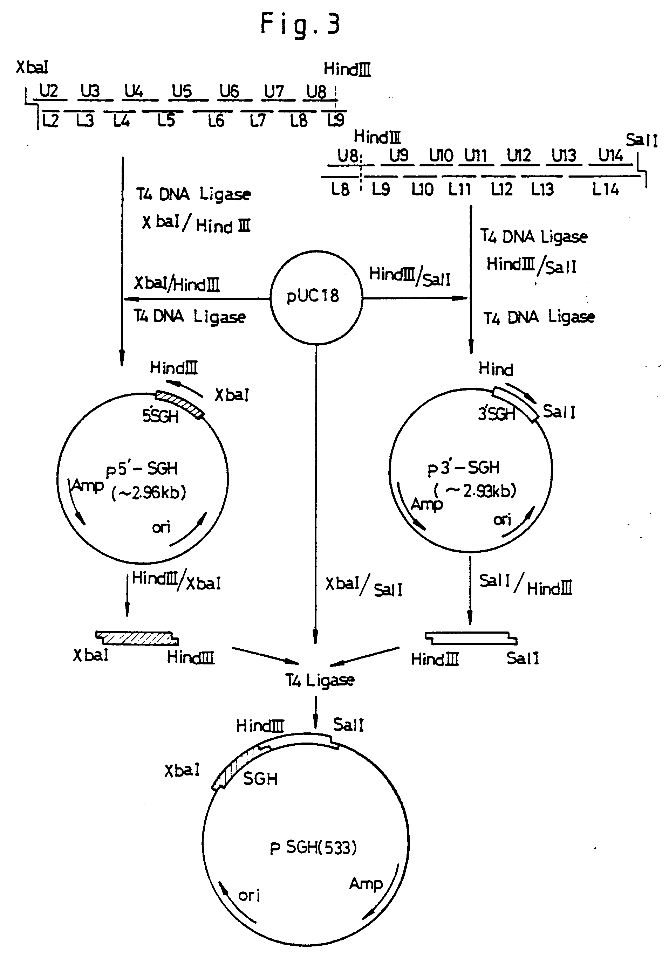

The synthetic oligonucleotides are ligated and inserted to a vector for E.coli,pUC18[Norrander, J. et al. Gene 36 (1983) 101] to produce pSGH(533) comprising an XbaI/SalI restriction fragment of 533 bp, which does not have an N-terminal region of the complete salmon growth hormone gene (Refer to Fig.3).

A method for the production of salmon growth hormone in yeast cells is as follows: the clone comprising the complete salmon growth hormone gene with a synthetic adaptor is ligated between the NcoI site and SalI site of a vector having a promoter and a terminator gene, pBS100 [Yeast 2.72 (1986) ; Chiron Corp.Emeryville,CA94608, U.S.A.] to express in yeast, wherein the adaptor is comprised of an NcoI restriction site, an initiation codon and some amino acid codons corresponding to the N-terminal region of salmon growth hormone. The obtained vector is pBS-SGH.

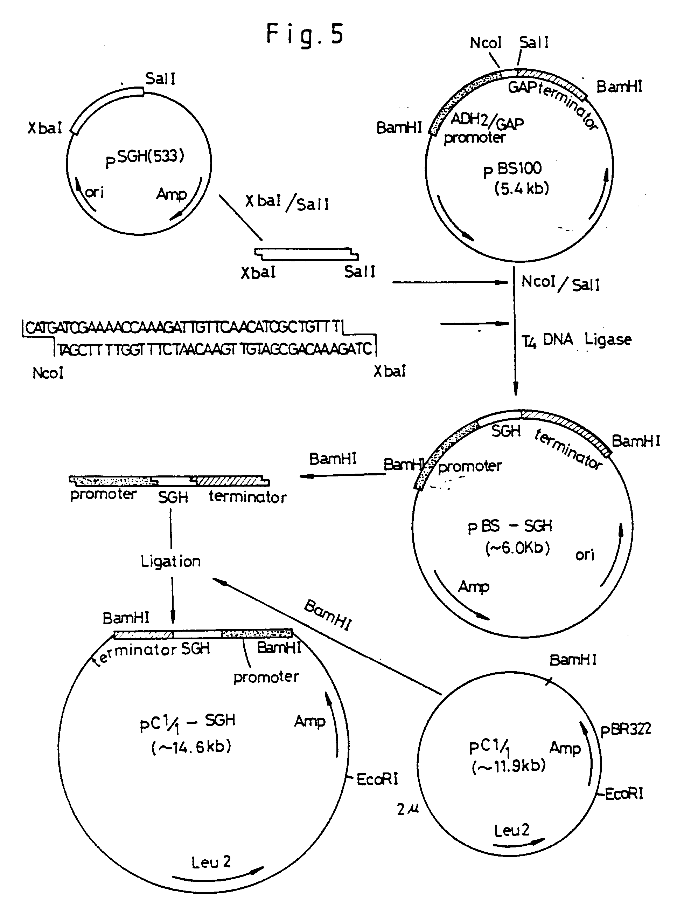

The vector pBS-SGH was treated with BamHI restriction enzyme to separate the BamHI fragment(about 2.7Kb) comprising promoter, salmon growth hormone gene and terminator. The BamHI fragment is inserted into the BamHI restriction site of a yeast expression vector,pC1/1, which is automatically replicated in E.coli and yeast[ATCC 37115; Brake et al. Proc.Natl.Acad. Sci USA 81(1984), 4642] to produce pC1/1-SGH (Refer to Fig.5).

The above yeast expression vector cloned to yeast strain, DCO4 [Broach,J.R. & Hicks,J.B. Cell 21, 501(1980); Yeast Genetic Stock Center, University of California, Berkeley, U. S. A.] by the method of Hinnen et al. The transformed yeast cell was cultured in YEPD medium comprising 2% glucose as in Example 4 for 48 hrs. When the concentration of glucose is exhausted, salmon growth hormone is induced automatically and salmon growth hormone (about 50mg per liter of culture at OD₆₅₀ = 15) was produced.

The invention is illustrated by the following Examples, without them limiting its range.

### Example 1: Ligation and cloning of synthetic oligonucleotides for salmon growth hormone gene

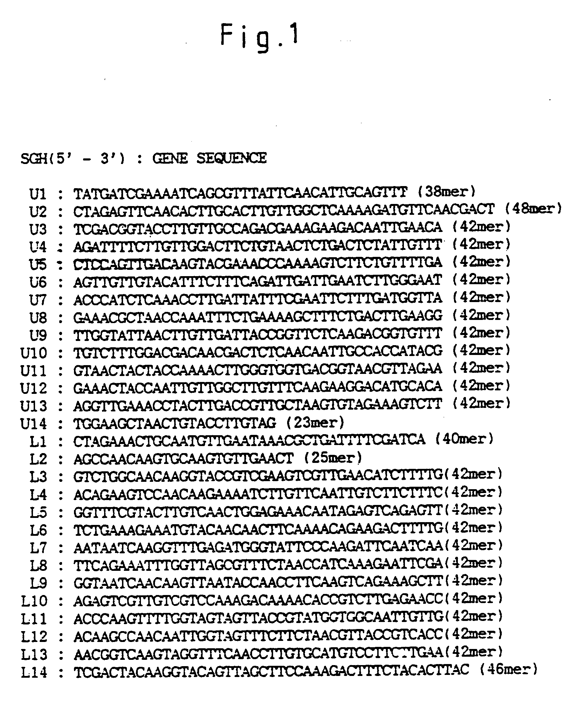

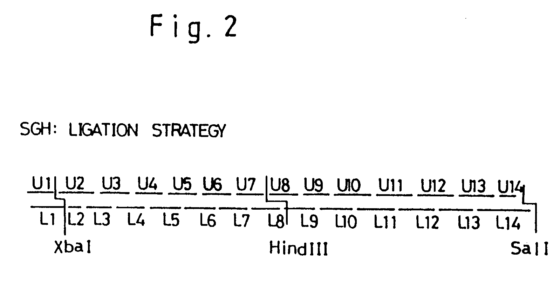

In order to obtain a complete salmon growth hormone gene from the synthetic oligonucleotides having the sequences of Fig.1, the ligation strategy of Fig.2 and vector pUC18 were used.

Each oligonucleotide(U8-U14/L9-L14) corresponding to the C-terminal HindIII and SalI restriction fragments of the salmon growth hormone gene was taken the amount of 0.05 OD₂₆₀ respectively, and then separately dried. Four units of T4 polynucleotide kinase are added to the total volume of 30 »l in the presence of 50mM Tris-HCl (pH 7.5), 1mM ATP, 1mM DTT and 10mM MgCl₂ and they are reacted for 30 minutes in order to phosphorylate the 5'-end residue of the oligonucleotides.

After the oligonucleotides are pooled and treated with an equal volume of phenol and chloroform mixture (24:1, v/v), they are precipitated with ethanol. The precipitates were dissolved in 53 »l of a buffer solution comprising 60mM Tris-HCl (pH 7.5), 1mM DTT and 10mM MgCl₂. The solution was placed in 95 ° C water bath and kept at room temperature for 6 hrs until the temperature drops to room temperature.

Twenty units of T4 DNA ligase and 5 »l of 10 mM ATP were added herein and ligation reaction was done at room temperature for 10 mins. This reaction solution was treated with phenol and chloroform mixture and precipitated with ethanol as described above.

Ten units each of HindIII and SalI restriction enzymes are added to the precipitated in the presence of a buffer solution comprising 60 mM Tris-HCl (pH 7.6), 10mM MgCl₂ and 100 mM NaCl and reacted at 37 ° C for 1 hr.

After 7% polyacrylamide gel electrophoresis, a band corresponding to 220-300 base pairs is cut from the gel. After electroelution, the precipitates were dissolved in 20 »l of distilled water.

Three »l of DNA and 10 ng of a vector, pUC18, cut with HindIII and SalI restriction enzymes are ligated in the presence of the ligation solution comprising 60 mM Tris-HCl (pH 7.5), 10 mM MgCl₂, 1 mM ATP and 10 units of T4 DNA ligase at 14 ° C for 16 hrs.

E.coli JM 103[BRL,U.S.A., Messing,J. Methods in Enzymology, 101, 20 (1983)] competent cell was added to the ligation mixture and transformed according to Hanahan's method [J.Mol.Biol 118, 557(1983)] at 37 ° C overnight.

The recombinant vector, p3'-SGH was selected from the white colonies according to Birnboim and Doly's method [Nucleic Acid Res.7, 1513 (1979)].

The oligonucleotides(U1-U7/L2-L8) corresponding to the N-terminal XbaI and HindIII restriction fragments of the synthetic oligonucleotides were ligated by the same method as mentioned above and inserted to pUC18, cut with XbaI and HindIII restriction enzymes to bet p5'-SGH by the method of Fig.3.

Finally, the DNA sequences of p3'-SGH and p5'-SGH are confirmed by the method of Sanger's dideoxy chain termination method [Proc. Natl. Acad. Sci. USA 74, 5473, (1977)] (Refer to Fig. 4).

The HindIII/SalI restriction fragment from p3'-SGH and the XbaI/HindIII restriction fragment from p5'-SGH are isolated respectively and ligated to a vector, pUC18, cut with XbaI and SalI restriction enzymes to produce pSGH(533) (Refer to Fig.3).

### Example 2: Manipulation of synthetic salmon growth hormone gene for expression in yeast cells

The 5'-end deficient part of the complete salmon growth hormone gene exists in pSGH(533) and it was synthesized by a DNA synthesizer. In the synthetic adaptor, the NcoI restriction enzyme site, an initiation codon and codons corresponding to NH₂-terminal 12 amino acids of salmon growth hormone were synthesized by selecting codons preferentially used in yeast cells (Refer to Fig. 5).

The process of cloning is as follows; pSGH(533) was treated with XbaI and SalI restriction enzymes to obtain a restriction fragment corresponding to 533 bp and the fragment was separated through agarose electrophoresis.

The synthetic adaptor was phosphorylated with T4 polynucleotide kinase as described previously. One »l of the phosphorylated adaptor reactant, 3 »l(30 ng) of the XbaI/SalI fragment of growth hormone gene and 1 »l (7 ng) of vector pBS100 (Yeast 2, 72(1986)) cut with NcoI and SalI restriction enzymes were mixed together in the presence of 2 »l of 10 mM ATP, 2 »l of 10-fold concentrated ligation reaction buffer solution, 1 »l of ligase and 10 »l of distilled water and incubated at 14 ° C for 16 hrs. As mentioned in sample 1, the ligation mixture was cloned to E.coli cell HB 101 (ATCC 33694) and a recombinant vector, pBS-SGH comprising a promoter, the complete salmon growth hormone gene and a terminator for expression in yeast was obtained.

The above mentioned vector, pBS100, contains the hybrid promoter of alcohol dehydrogenase II promoter and glyceraldehyde 3'-phosphate dehydrogenase promoter.

The vector, pBS-SGH was treated with BamHl restriction enzyme to separate the BamHI restriction fragment of about 2,700 bp, comprising a hybrid promoter, the salmon growth hormone gene and the terminator of glyceraldehyde 3'-phosphate dehydrogenase. The BamHI restriction fragment was inserted into the BamHI restriction site of the vector for yeast expression, pC1/1(ATCC 37115), to obtain pC1/1-SGH (Refer to Fig.5).

Ten »g of pC1/1-SGH gene was cloned to yeast strain DCO4 [Yeast Genetic Stock Center, University of California, Berkeley, U.S.A., Broach, J.R. & Hicks, J.B. Cell 21, 501 (1980)] according to Hinnen's method [Proc. Natl. Acad. Sci. USA 75 (1978), 1929] and plated on agar-plates without leucine, comprising 6.7g of Yeast Nitrogen Base without amino acids, 182 g of sorbitol 2% glucose, 0.25 g of Leu-supplements and 20 g of Bactoagar per liter.

Recombinant clones containing salmon growth hormone gene were observed 3 to 5 days after transformation.

### Example 3: Cultivation for producing salmon growth hormone in yeast cells and its identification

Each 3 ml of yeast cells transformed with vector pC1/1-SGH was cultured in a culture medium without leucine (6.7 g of Yeast Nitrogen Base without amino acids,0.25 g of Leu-supplements and 6 % glucose per liter of culture medium) at 30 ° C for 24 hrs. YEPD culture medium(100ml) comprising 2% peptone, 1% yeast extract and 2% glucose was inoculated with 1 ml of the overnight culture and cultured at 30 ° C for 24hrs. 40ml of ethanol was added thereto and the solution was further cultured for 24 hrs.

The resultant OD₆₅₀ was about 40. That OD₆₅₀ per ml of 10 was taken and centrifuged. It was dissolved in 400 »l of a buffer solution containing 10 mM Tris-HCl (pH 7.5), 1 mM EDTA, 2 mM phenylmethylsulfonyl fluoride and 8 M urea. The same volume of glass beads with a diameter of 0.45 mm was added thereto and shaken vigorously. After rupturing the cell walls and allowing the salmon growth hormone to elute into the buffer solution, 4 »l of eluted solution was examined by electrophoresis on 12.5% SDS polyacrylamide gel.

For the purpose of comparison, protein from Bio-Rad Company was run in one lane, and all the proteins of yeast cells transformed with vector pC1/1, without the salmon growth hormone gene, in another. In the lane that represents all the proteins of yeast cells transformed with vector pC1/1-SGH, with the salmon growth hormone gene, gel scanning shows that the salmon growth hormone appears in a band, at about 22 kD, in an amount corresponding to 5% of the total proteins. Peptide sequencing of the purified salmon growth hormone showed that it was mature salmon growth hormone, with an N-terminal isoleucine.

## Claims

1. A method for the production of salmon growth hormone, comprising (a) synthesising oligonucleotides having XbaI, HindIII and SalI restriction sites as shown in Fig. 4 and corresponding to the amino-acid sequence of salmon growth hormone, (b) restricting the oligonucleotides to form XbaI/HindIII and HindIII/SalI fragments, (c) ligating the fragments into respective vectors for E. coli, (d) isolating the said fragments from the respective vectors, (e) ligating the fragments to a vector for E. coli, to make a partial salmon growth hormone gene, lacking a portion of the N-terminus, (f) inserting the partial salmon growth hormone gene and a N-terminal synthetic adaptor into a vector for E. coli consisting of a hybrid alcohol dehydrogenase II/glyceraldehyde 3'-phosphate dehydrogenase promoter and a terminator, to obtain a cassette of promoter-salmon growth hormone gene-terminator, (g) inserting the cassette into an E. coli-yeast shuttle vector for expression in yeast, and (h) expressing the resultant vector in yeast cells.

2. A method according to claim 1, wherein the N-terminal synthetic adaptor is the following nucleotide sequence, with NcoI and XbaI restriction sites:

5' CATGATCGAAAACAAAAGATTGTTCAACATTGCTGTTT 3'
3' TAGCTTTTGTTTCTAACAAGTTGTAACGACAAAGATC 5'
NcoI site | XbaI site

3. A method according to claim 1 or claim 2, wherein the synthetic adaptor was synthesized by amino acid codons which are preferentially used in yeast.

## Patentansprüche

1. Verfahren zur Herstellung von Lachs-Wachstumshormon, welches umfaßt
(a) die Synthese von Oligonukleotiden, die XbaI-, HindIII- und SalI-Restriktionsstellen wie in Fig. 4 gezeigt aufweisen und mit der Aminosäuresequenz von Lachs-Wachstumshormon korrespondieren,
(b) Schneiden der Oligonukleotide, um XbaI/HindIII- und HindIII/SalI-Fragmente zu erzeugen,
(c) Ligieren der Fragmente in die jeweiligen Vektoren für E. coli,
(d) Isolierung der Fragmente aus den jeweiligen Vektoren,
(e) Ligieren der Fragmente in einen Vektor für E. coli, um ein partielles Lachs-Wachstumshormon-Gen zu erzeugen, dem ein Teil des N-Terminus fehlt,
(f) Insertion des partiellen Lachs-Wachstumshormon-Gens und eines N-terminalen synthetischen Adaptors in einen Vektor für E. coli, der aus einem hybriden Alkoholdehydrogenase II/Glyceraldehyd 3'-Phosphat-dehydrogenase-Promotor und einem Terminator besteht, um eine Kassette von Promotor--Lachs-Wachstumshormon-Gen--Terminator zu erhalten,
(g) Insertion der Kassette in einen E. coli-Hefe-"Shuttle"-Vektor zur Expression in Hefe, und
(h) Expression des resultierenden Vektors in Hefezellen.

2. Verfahren nach Anspruch 1, worin der N-terminale synthetische Adaptor die folgende Nukleotidsequenz mit NcoI- und XbaI-Restriktionsstellen aufweist:

5' - CATGATCGAAAACAAAAGATTGTTCAACATTGCTGTTT - 3'
——                                                                                    ——
3' - TAGCTTTTGGTTTCTAACAAGTTGTAACGACAAAGATC - 5'

NcoI-Stelle                                                                    XbaI-Stelle

3. Verfahren nach Anspruch 1 oder 2, worin der synthetische Adaptor durch Aminosäurekodons synthetisiert wurde, die vorzugsweise in Hefe verwendet werden.

## Revendications

1. Procédé de production d'une hormone de croissance de saumon, comprenant (a) la synthèse d'oligonucléotides ayant des sites de restriction XbaI, HindIII et SalI comme cela est montré sur la figure 4 et correspondant à la séquence d'acides aminés de l'hormone de croissance de saumon, (b) la coupure des oligonucléotides pour former des fragments XbaI/HindIII et HindIII/SalI, (c) la ligature des fragments dans des vecteurs respectifs de E. coli, (d) l'isolement desdits fragments à partir des vecteurs respectifs, (e) la ligature des fragments à un vecteur de E. coli pour fabriquer un gène partiel d'hormone de croissance de saumon qui est dénué d'une partie de la terminaison N, (f) l'insertion du gène partiel de l'hormone de croissance de saumon et d'un adaptateur artificiel à terminaison N dans un vecteur de E. coli constitué d'un promoteur hybride alcooldéshydrogénase II/glycéraldéhyde-3'-phosphate-déshydrogénase et d'un terminateur pour obtenir une cassette de promoteur-gène d'hormone de croissance de saumon-terminateur, (g) l'insertion de la cassette dans un vecteur navette de E. coli-levure destiné à une expression dans une levure et (h) l'expression du vecteur résultant dans des cellules de levure.

2. Procédé selon la revendication 1, dans lequel l'adaptateur artificiel à terminaison N est constitué de la séquence de nucléotides suivante ayant les sites de restriction NcoI et XbaI:

5' – CATGATCGAAAACCAAAGATTGTTCAACATCGCTGTTT – 3'
3' – TAGCTTTTGGTTTCTAACAAGTTGTAGCGACAAAGATC – 5'
Site NcoI Site XbaI

3. Procédé selon la revendication 1 ou la revendication 2, dans lequel on a synthétisé l'adaptateur artificiel par des codons d'acides aminés qu'on a utilisés de préférence dans une levure.
